# EUROPEAN PATENT APPLICATION

(11) **EP 4 029 426 A1**
(43) Date of publication of application: **20.07.2022**
(21) Application number: 20863739.7
(22) Date of filing: 27.08.2020
(51) Int. Cl.: A61B 3/028

(54) **INFORMATION ANALYZING DEVICE AND INFORMATION ANALYZING PROGRAM**

(30) Priority: 12.09.2019 JP 2019166005
(71) Applicant: Nidek Co., Ltd., Gamagori-shi, Aichi, 443-0038 (JP)
(72) Inventor: HIRAYAMA, Yukito, Gamagori-shi Aichi 443-0038 (JP)
(74) Representative: Hoefer & Partner Patentanwälte mbB
(86) International application number: PCT/JP2020/032472
(87) International publication number: WO 2021/049314

(57) **Abstract**

This information analyzing device analyzes information relating to a subjective examination and comprises: a subjective value acquisition means for acquiring a subjective value of an eye under examination measured in the subjective examination; a data log acquisition means for acquiring a data log relating to the subjective examination when the subjective value of the eye under examination is measured in the subjective examination; an evaluation information acquisition means for acquiring evaluation information for evaluating the reliability of the subjective examination on the basis of the data log; and an output means for outputting the subjective value and the evaluation information. This information analyzing device makes it easy for an examiner to determine the suitability of a subjective examination carried out by an inexperienced operator, thereby facilitating determination of the necessity of a re-examination of an eye under examination, a suitable prescription for an eye under examination, and the like.

## Description

### TECHNICAL FIELD

The present disclosure relates to an information analyzing device and an information analyzing program for analyzing information related to a subjective examination.

### BACKGROUND ART

As an example of a device that performs a subjective examination on a subject eye, a subjective optometry device that subjectively measures optical characteristics of the subject eye by disposing an optical member in front of the subject eye and presenting a visual target via the optical member (refer to Patent Literature 1).

### CITATION LIST

### PATENT LITERATURE

Patent Literature 1: JP-A-H05-176893

### SUMMARY OF INVENTION

Incidentally, in the subjective examination, an unfamiliar operator may operate the subjective optometry device to perform a subjective examination on the subject eye and acquire a measurement result. In this case, the subjective examination by the operator did not always proceed well, and when the examiner used the measurement result from the operator as it was, the prescription may be inappropriate for the subject eye.

In view of the above-described related art, a technical subject of the present disclosure is to provide an information analyzing device and an information analyzing program that can easily determine the suitability of a subjective examination using a subjective optometry device.

According to a first aspect of the present disclosure, there is provided an information analyzing device that analyzes information related to a subjective examination, the information analyzing device including:
a subjective value acquisition means for acquiring a subjective value of a subject eye measured by the subjective examination;
a data log acquisition means for acquiring a data log related to the subjective examination in a case where the subjective value of the subject eye is measured by the subjective examination;
an evaluation information acquisition means for acquiring evaluation information for evaluating reliability of the subjective examination, based on the data log; and
an output means for outputting the subjective value and the evaluation information.

According to a second aspect of the present disclosure, there is provided an information analyzing program used in an information analyzing device that analyzes information related to a subjective examination, the program being executed by a processor of the information analyzing device to cause the information analyzing device to execute:
a subjective value acquiring step of acquiring a subjective value obtained by measuring optical characteristics of a subject eye by the subjective examination;
a data log acquiring step of acquiring a data log related to the subjective examination; and
an evaluation information acquiring step used to confirm reliability of the subjective examination, based on the data log.

### BRIEF DESCRIPTION OF DRAWINGS

[FIG. 1] FIG. 1 is a view showing an example of a flow of a subjective examination on a subject eye.
[FIG. 2] FIG. 2 is an external view of an optometry device.
[FIG. 3] FIG. 3 is a view showing a measurement unit of the optometry device.
[FIG. 4] FIG. 4 is a schematic configuration view of the inside of the optometry device when viewed from the front.
[FIG. 5] FIG. 5 is a schematic configuration view of the inside of the optometry device when viewed from the side.
[FIG. 6] FIG. 6 is a schematic configuration view of the inside of the optometry device when viewed from above.
[FIG. 7] FIG. 7 is a view showing a control system of the optometry device.
[FIG. 8] FIG. 8 is an example of a flowchart showing a control operation of the optometry device.
[FIG. 9] FIG. 9 is an example of a flowchart showing acquisition of evaluation values for subjective measurement by an assistant.

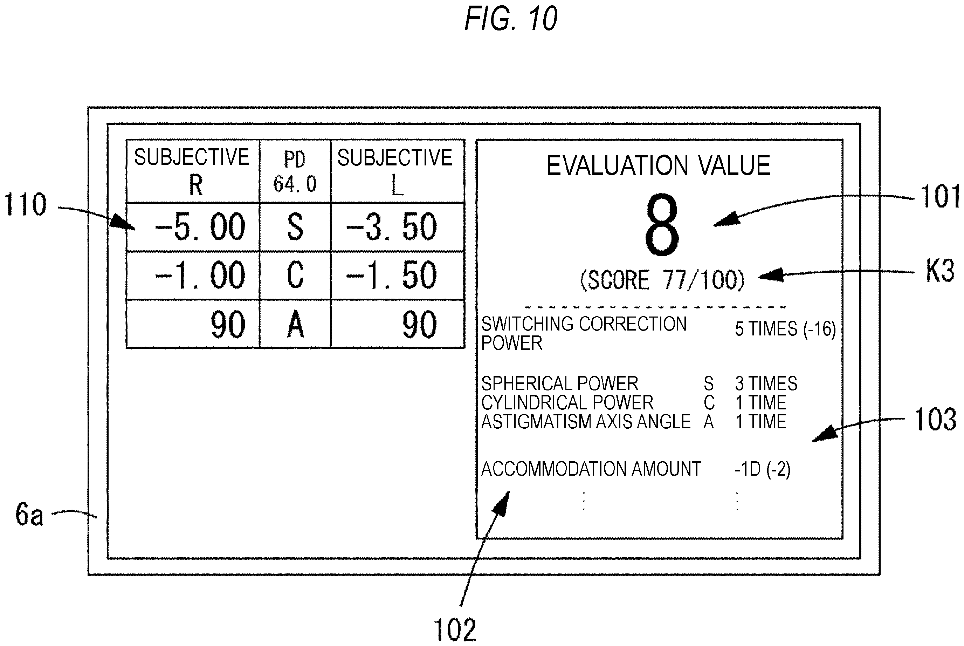

[FIG. 10] FIG. 10 is an example of a display screen of a monitor.

### DESCRIPTION OF EMBODIMENTS

### <Overview>

The overview of the information analyzing device according to an embodiment of the present disclosure will be described. The items classified by < > below can be used independently or in association with each other.

### <Subjective value acquisition means>

An information analyzing device in the present embodiment includes a subjective value acquisition means (for example, control unit 70). The subjective value acquisition means acquires a subjective value of a subject eye measured by the subjective examination on the subject eye. For example, the subjective value of the subject eye may be a completely corrected value of the subject eye. For example, the completely corrected value of the subject eye may be a value of the most positive correction power at which the highest visual acuity of the subject eye can be obtained. Further, for example, the subjective value of the subject eye may be a prescription value used when prescribing spectacles to the subject eye. For example, the prescription value of the subject eye may be a value of the correction power at which a predetermined visual acuity of the subject eye can be obtained.

For example, the subjective value acquisition means may acquire the subjective value of the subject eye by receiving the subjective value measured by a subjective optometry device (for example, refer to JP-A-2017-086652) which subjectively measures the optical characteristics of the subject eye by projecting a visual target light flux toward the subject eye and changing the optical characteristics of the visual target light flux. When the information analyzing device includes a subjective measurement means which will be described later, the subjective value acquisition means may acquire the subjective value of the subject eye by measuring the subjective value using the subjective measurement means.

### <Data log acquisition means>

The information analyzing device in the present embodiment includes a data log acquisition means (for example, control unit 70). The data log acquisition means acquires a data log related to the subjective examination in a case where the subjective value of the subject eye is measured by the subjective examination on the subject eye.

For example, the data log acquisition means may acquire the data log related to the subjective examination by receiving the data log in a case where the subjective value is measured by the subjective optometry device. When the information analyzing device includes the subjective measurement means which will be described later, the data log acquisition means may acquire the data log in a case where the subjective value is measured by using the subjective measurement means.

The data log related to the subj ective examination on the subject eye may be managed in association with the identification information (for example, ID number or the like) for identifying examinee. In this case, the data log acquisition means can acquire the data log related to the subjective examination in a case where the subjective value of the subject eye is measured, by extracting the data log to which the predetermined identification information is added.

In the present embodiment, the data log related to the subjective examination may be a log obtained by performing the subjective examination.

For example, the data log related to the subjective examination may be an operation data log related to the operation of the operator in a case where the operator performs the subjective examination.

For example, the operation data log of the operator may include an operation data log related to the operation of a visual target presenting means by the operator. As an example, a data log indicating at least one of the number of times (frequency) of switching a visual target presented to the subject eye by the operator, the type of the visual target presented to the subject eye by the operator, and the like may be included. The types of visual targets presented to the subject eye by the operator may include the number of types of visual targets presented to the subject eye by the operator, the order in which the visual targets corresponding to each visual acuity value are presented to the subject eye by the operator, and the like.

Further, for example, the operation data log of the operator may include an operation data log related to the operation of a correction optical system by the operator. As an example, a data log indicating at least one of the number of times (frequency) of switching correction power obtained by correcting the subject eye by the operator, the correction power added to the subject eye by the operator, and the like may be included. The correction power added to the subject eye by the operator may include the number of correction powers added to the subject eye by the operator in a process of the subjective examination, the order of the correction power added to the subject eye by the operator in the process of the subjective examination, and the like.

Further, for example, the operation data log of the operator may include a data log indicating time required for the subjective examination by the examiner. As an example, a data log indicating the time required from the start to the end of the operation in the subjective examination by the operator (for example, the time required after a signal that the operator selected a visual target to be presented to the subject eye is emitted until a signal that the operator determined the subjective value of the subject eye is emitted) may be included.

When the subjective examination on the subject eye is automatically executed, the data log acquisition means may acquire the operation data log by the control means that has executed the subjective examination, as the data log related to the subjective examination.

For example, in this case, the operation data log related to the operation of the visual target presenting unit means by the control means may be included. As an example, there is a data log indicating at least one of the number of times (frequency) of switching the visual target by the control means, the type of visual target switched by the control means, and the like. Further, for example, in this case, an operation data log related to the operation of the correction optical system by the control means may be included. As an example, there is a data log indicating at least one of the number of times (frequency) of switching the correction power by the control means, the correction power added to the subject eye by the control means, and the like. Further, for example, in this case, a data log indicating the time required for the subjective examination by the control means may be included. As an example, a data log indicating the time required from the start to the end of the subjective examination by the control means (for example, the time required after switching the visual target presented by the control means until storing the subjective value of the subject eye in storage means) may be included.

It is needless to say that the subj ective examination on the subject eye may be partially executed by an operator (examiner or examinee) and may be partially automatically executed. In this case, the data log acquisition means may acquire both the operation data log related to the operation in a case where the operator performs the subjective examination and the operation data log by the control means that has executed the subjective examination.

Further, for example, the data log related to the subjective examination may be a data log indicating the state of the subject eye (change during the examination of the examinee) which is generated by the examinee undergoing the subjective examination. For example, the data log indicating the state of the subject eye (change during the examination of the examinee) may be a data log indicating at least one of the number of times (frequency) of squinting the subject eye, the number of times (frequency) of blinking the subject eye, the change in the visual acuity value corresponding to the visual target that the subject eye can visually confirm, the change in the pupil size of the subject eye, whether or not the subject eye is ptosis, the time required for the examinee to respond, and the like.

The data log acquisition means may be configured to acquire a data log including at least one of the above-described data logs as the data log related to the subjective examination. In other words, the data log acquisition means may acquire one data log in the above-described data log as the data log related to the subjective examination, or may acquire a plurality of data logs in the above-described data log. It is needless to say that the data log acquisition means may acquire a data log different from the above-described data log as the data log related to the subjective examination. For example, the data log acquisition means may acquire a data log indicating the subjective value obtained in the subjective examination, an error log generated in the subjective examination, and the like.

Further, the data log acquisition means may acquire a data log obtained in an examination different from the subjective examination. In this case, the data log acquisition means may acquire a data log indicating an objective value (for example, the objective refractive power of the subject eye and the aberration information of the subject eye) obtained in the objective examination, the reliability of the objective examination, and the like. In addition, the data log acquisition means may acquire a data log indicating the results of interviews (for example, age, gender, occupation, and purpose of making spectacles) conducted with the examinee, a data log indicating the power (that is, values of spectacles) of the spectacles worn by the examinee, and the like.

In the present embodiment, the data log acquisition means may acquire a data log related to a spherical examination for acquiring spherical information of the subject eye, as the data log related to the subjective examination. For example, the data log related to the spherical examination of the subject eye may be any data log obtained by performing the spherical examination. Accordingly, it is possible to obtain evaluation information which will be described later that reflects whether or not the spherical information of the subject eye is appropriately examined, and it becomes possible to easily determine reexamination or prescription for the subject eye.

For example, the data log related to the spherical examination may be an operation data log related to the operation of the operator in a case where the operator performs the spherical examination.

For example, the operation data log related to the spherical examination may include an operation data log related to the operation of the visual target presenting means in the spherical examination by the operator. As an example, a data log indicating at least one of the number of times of switching the visual target in the spherical examination, the type of the visual target presented in the spherical examination, and the like may be included.

Further, for example, the operation data log related to the spherical examination may include an operation data log related to the operation of the correction optical system in the spherical examination by the operator. As an example, a data log indicating at least one of the number of times of switching the spherical power in the spherical examination, the spherical power added to the subject eye in the spherical examination, and the like may be included.

Further, for example, the operation data log related to the spherical examination may include a data log indicating the time required for the spherical examination by the operator. As an example, a data log indicating the time required from the start to the end of the operation in the spherical examination by the operator may be included.

It is needless to say that, for example, the data log related to the spherical examination may include a data log indicating the state of the subject eye which was generated by the examinee undergoing the spherical examination, a data log indicating the examination result obtained in the spherical examination (for example, the spherical power of the subject eye and the equivalent spherical power of the subject eye), an error log generated in the spherical examination, and the like.

The data log acquisition means may acquire at least a data log related to an accommodation examination for acquiring accommodation information of the subject eye, as the data log related to the spherical examination. For example, the data log related to the accommodation examination of the subject eye may be any data log obtained by performing the accommodation examination. By using such a data log, it is possible to obtain evaluation information which will be described later that reflects the accommodation information of the subject eye, and it becomes possible to easily perform an appropriate examination and prescription for the subject eye.

For example, the data log related to the accommodation examination may be an operation data log related to the operation of the operator in a case where the operator performs the accommodation examination.

For example, the operation data log related to the accommodation examination may include an operation data log related to the operation of the visual target presenting means in the accommodation examination by the operator. As an example, a data log indicating at least one of the number of times of switching the visual target in the accommodation examination, the type of the visual target presented in the accommodation examination, and the like may be included.

Further, for example, the operation data log related to the accommodation examination may include an operation data log related to the operation of the correction optical system in the accommodation examination by the operator. As an example, a data log indicating at least one of the number of times of switching the spherical power in the accommodation examination, the spherical power added to the subject eye in the accommodation examination, and the like may be included.

Further, for example, the operation data log related to the accommodation examination may include a data log indicating the time required for the accommodation examination by the operator. As an example, a data log indicating the time required from the start to the end of the operation in the accommodation examination by the operator may be included.

It is needless to say that, for example, the data log related to the accommodation examination may include a data log indicating the state of the subject eye which was generated by the examinee undergoing the accommodation examination, a data log indicating the examination result obtained in the accommodation examination, an error log generated in the accommodation examination, and the like.

The data log indicating the examination result obtained in the accommodation examination may include information based on a plurality of optical characteristics of the subject eye, information related to accommodation of the subject eye, and the like. As an example, the information based on the plurality of optical characteristics of the subject eye may be information such as the difference and average value of each subjective value obtained at the start and end of the accommodation examination, and the difference and average value of the objective value by the objective examination and the subjective value by the subjective examination. Further, as an example, the information related to the accommodation of the subject eye may be information such as the presence or absence of accommodation, the accommodation amount, and the like.

In addition, in the present embodiment, the data log acquisition means may acquire a data log related to an astigmatism examination for acquiring astigmatism information of the subject eye, as the data log related to the subjective examination. For example, the data log related to the astigmatism examination of the subject eye may be any data log obtained by performing the astigmatism examination. Accordingly, it is possible to obtain evaluation information which will be described later that reflects whether or not the astigmatism information of the subject eye is appropriately examined, and it becomes possible to easily determine reexamination or prescription for the subject eye.

For example, the data log related to the astigmatism examination may be an operation data log related to the operation of the operator in a case where the operator performs the astigmatism examination.

For example, the operation data log related to the astigmatism examination may include an operation data log related to the operation of the visual target presenting means in the astigmatism examination by the operator. As an example, a data log indicating at least one of the number of times of switching the visual target in the astigmatism examination, the type of the visual target presented in the astigmatism examination, and the like may be included.

Further, for example, the operation data log related to the astigmatism examination may include an operation data log related to the operation of the correction optical system in the astigmatism examination by the operator. As an example, a data log indicating at least one of the number of times of switching the astigmatic power (cylindrical power and astigmatism axis angle) in the astigmatism examination, the astigmatic power (cylindrical power and astigmatism axis angle) added to the subject eye in the astigmatism examination, and the like may be included.

Further, for example, the operation data log related to the astigmatism examination may include a data log indicating the time required for the astigmatism examination by the operator. As an example, a data log indicating the time required from the start to the end of the operation in the astigmatism examination by the operator may be included.

It is needless to say that, for example, the data log related to the astigmatism examination may include a data log indicating the state of the subject eye which was generated by the examinee undergoing the astigmatism examination, a data log indicating the examination result obtained in the astigmatism examination (for example, the cylindrical power of the subject eye and the astigmatism axis angle of the subject eye), an error log generated in the astigmatism examination, and the like.

For example, the data log acquisition means may acquire at least a data log related to a residual astigmatism examination for acquiring residual astigmatism information of the subject eye, as the data log related to the astigmatism examination. For example, the data log related to the residual astigmatism examination of the subject eye may be any data log obtained by performing the residual astigmatism examination. By using such a data log, it is possible to obtain evaluation information which will be described later that reflects the residual astigmatism information of the subject eye, and it becomes possible to easily perform an appropriate examination and prescription for the subject eye.

For example, the data log related to the residual astigmatism examination may be an operation data log related to the operation of the operator in a case where the operator performs the residual astigmatism examination.

For example, the operation data log related to the residual astigmatism examination may include an operation data log related to the operation of the visual target presenting means in the residual astigmatism examination by the operator. As an example, a data log indicating at least one of the number of times of switching the visual target in the residual astigmatism examination, the type of the visual target presented in the residual astigmatism examination, and the like may be included.

Further, for example, the operation data log related to the residual astigmatism examination may include an operation data log related to the operation of the correction optical system in the residual astigmatism examination by the operator. As an example, a data log indicating at least one of the number of times of switching the astigmatic power in the residual astigmatism examination (that is, the number of times of correcting the residual astigmatism), the astigmatic power added to the subject eye in the residual astigmatism examination, and the like may be included.

Further, for example, the operation data log related to the accommodation examination may include a data log indicating the time required for the accommodation examination by the operator. As an example, a data log indicating the time required from the start to the end of the operation in the accommodation examination by the operator may be included.

It is needless to say that, for example, the data log related to the residual astigmatism examination may include a data log indicating the state of the subject eye which was generated by the examinee undergoing the residual astigmatism examination, a data log indicating the examination result obtained in the residual astigmatism examination, an error log generated in the residual astigmatism examination, and the like.

The data log indicating the examination results obtained in the residual astigmatism examination may include information related to the residual astigmatism of the subject eye, and the like. As an example, the information related to the residual astigmatism of the subject eye may be at least one of information such as the presence or absence of residual astigmatism, and the convergence degree of residual astigmatism. For example, the convergence degree of residual astigmatism may indicate whether or not the residual astigmatism has gradually disappeared by correcting the residual astigmatism of the subject eye.

In the present embodiment, the data log acquisition means may be configured to acquire at least one of the data log related to the spherical examination and the data log related to the astigmatism examination, as the data log related to the subjective examination.

### <Evaluation information acquisition means>

The information analyzing device in the present embodiment includes an evaluation information acquisition means (for example, control unit 70). The evaluation information acquisition means acquires evaluation information for evaluating the reliability of the subjective examination, based on the data log acquired by the data log acquisition means. For example, the reliability of the subjective examination may indicate whether or not the subjective examination by the operator has been appropriately carried out. As an example, the reliability may indicate whether the operator has carried out the subjective examination in a correct procedure, whether the items of the subjective examination by the operator have been sufficient, and the like.

The evaluation information acquisition means acquires information that allows the operator to evaluate the reliability of the subjective examination, as the evaluation information. For example, the operator may acquire at least one of an evaluation symbol, an evaluation value, an evaluation color, an evaluation graph, and the like for evaluating the reliability of the subjective examination. It is needless to say that the evaluation information acquisition means may be configured to acquire a plurality of information among information such as the evaluation symbol, the evaluation value, the evaluation color, the evaluation graph, and the like.

For example, the evaluation information acquisition means may acquire a symbol indicating the suitability of the subjective examination, based on the data log, as an evaluation symbol indicating the evaluation information. As an example, a symbol indicating that the subjective examination is appropriate, a symbol indicating that the subjective examination is inappropriate, and the like may be acquired.

Further, for example, the evaluation information acquisition means may acquire evaluation values classified based on the data log related to the subjective examination, as an evaluation value indicating the evaluation information, based on the data log. As an example, the evaluation values may be indicated by symbols such as an evaluation value A, an evaluation value B, ..., and an evaluation value n, which are classified based on the data log related to the subjective examination. Further, as an example, the evaluation values may be indicated by numerical values (that is, n-step numerical values) such as the evaluation value 1, the evaluation value 2, ..., and the evaluation value n, which are classified based on the data log related to the subj ective examination. The number of classifications based on the data log (here, the number of symbols and the number of steps) may be set to any number.

Further, for example, the evaluation information acquisition means may acquire an evaluation color indicating the evaluation information, based on the data log. As an example, the evaluation information may be color-coded according to whether or not a predetermined data log is included in the data log related to the subjective examination. For example, the correspondence between the evaluation of the reliability of the subjective examination and the color coding of the evaluation information may be set in advance.

Further, for example, the evaluation information acquisition means may acquire an evaluation graph indicating the evaluation information, based on the data log. For example, an evaluation graph associated with the high (or low) reliability of the subjective examination may be indicated for each item included in the data log related to the subjective examination.

For example, the evaluation information acquisition means may acquire evaluation information for evaluating the reliability of the subjective examination by using a table in which the data log related to the subjective examination of the subject eye and the evaluation information are associated with each other. For example, the table may be preset from experiments, simulations, and the like. In addition, in the present embodiment, the evaluation information acquisition means may acquire evaluation information for evaluating the reliability of the subjective examination by using an arithmetic expression in which the data log related to the subjective examination of the subject eye and the evaluation information are associated with each other. For example, the arithmetic expression may be preset from experiments, simulations, and the like. For example, when a predetermined data log is included in the data log related to the subjective examination, the evaluation information acquisition means may obtain the evaluation information by changing a table value, a coefficient of an arithmetic expression, and the like, based on the parameters of the predetermined data log.

The evaluation information acquisition means may be configured to acquire the evaluation information as information for evaluating the overall reliability of the subjective examination by the operator. In addition, the evaluation information acquisition means may be configured to acquire the evaluation information as information for evaluating at least partial reliability of the subjective examination by the operator. For example, in this case, reliability information may be acquired for each item of the subjective examination by the operator. It is needless to say that the evaluation information acquisition means may acquire the evaluation information as respective information for evaluating the overall reliability of the subjective examination by the operator and the reliability of each item of the subjective examination by the operator.

In the present embodiment, the evaluation information acquisition means may acquire evaluation information based on the data log related to the spherical examination, as the data log related to the subjective examination. In other words, the evaluation information acquisition means may acquire the evaluation information based on a data log indicating at least one of the number of times of switching the visual target in the spherical examination, the type of the visual target presented in the spherical examination, the number of times of switching the spherical power in the spherical examination, and the spherical power added to the subject eye in the spherical examination. For example, when the data log related to the accommodation examination is used as the data log related to the spherical examination, the evaluation information acquisition means may acquire the evaluation information based on a data log indicating at least one of the number of times of switching the visual target in the accommodation examination, the type of the visual target presented in the accommodation examination, the number of times of switching the spherical power in the accommodation examination, and the spherical power added to the subject eye in the accommodation examination.

In addition, in the present embodiment, the evaluation information acquisition means may acquire evaluation information based on the data log related to the astigmatism examination, as the data log related to the subjective examination. In other words, the evaluation information may be acquired based on a data log indicating at least one of the number of times of switching the visual target in the astigmatism examination, the type of the visual target presented in the astigmatism examination, the number of times of switching the astigmatic power in the astigmatism examination, and the astigmatic power added to the subject eye in the astigmatism examination. For example, when the data log related to the residual astigmatism examination is used as the data log related to the astigmatism examination, the evaluation information acquisition means may acquire the evaluation information based on at least one of the number of times of switching the visual target in the residual astigmatism examination, the type of the visual target presented in the residual astigmatism examination, the number of times of switching the astigmatic power in the residual astigmatism examination, and the astigmatic power added to the subject eye in the residual astigmatism examination.

In the present embodiment, the evaluation information acquisition means may be configured to acquire the evaluation information based on at least one of the above-described data log related to the spherical examination and the above-described data log related to the astigmatism examination.

It is needless to say that, in the present embodiment, the evaluation information acquisition means may acquire the evaluation information based on the data log indicating a state of the subject eye which was generated by the examinee undergoing the subjective examination, the data log indicating the subjective value obtained in the subjective examination, the error log generated in the subjective examination, and the like, as the data log related to the subjective examination.

### <Output means>

The information analyzing device in the present embodiment includes an output means (for example, control unit 70). The output means outputs the subjective value of the subject eye measured by the subjective examination and the evaluation information for evaluating the reliability of the subjective examination. In other words, the output means outputs the subjective value acquired by the subjective value acquisition means and the evaluation information acquired by the evaluation information acquisition means.

The output means may output the evaluation information itself as the evaluation information. For example, the output means may output the symbol indicating the suitability of the subjective examination, the evaluation value, and the like, as the evaluation information. Further, the output means may output a graph or the like based on the evaluation value, as the evaluation information. For example, the output means may output a color-coded graph or the like based on the evaluation value.

For example, the output means may output the subjective value and the evaluation information by displaying these on a display means (for example, monitor 6a), by generating a voice guide, by transmitting these to a memory, a server, or the like, by printing these to a printer or the like, by transmitting these to the external device, and the like. In addition, for example, the output means may output an identifier associated with the subjective value and the evaluation information. For example, in this case, a character string, a one-dimensional code (bar code and the like), a two-dimensional code (QR code (registered trademark), color code, and the like) may be used as the identifier.

In the present embodiment, the information analyzing device may receive the subjective value of the subject eye in the subjective examination performed, for the subject eye, by using a device different from the information analyzing device, and the data log related to the subjective examination, to acquire these. In addition, in the present embodiment, the information analyzing device may acquire the subjective value of the subject eye in the subjective examination performed, for the subject eye, by using the information analyzing device, and the data log related to the subjective examination. In this case, the information analyzing device can acquire the subjective value and the data log by providing the following subjective measurement means.

### <Subjective measurement means>

The information analyzing device may include a subjective measurement means (for example, subjective measurement optical system 25). The subjective measurement means subjectively measures the optical characteristics of the subject eye by projecting the visual target light flux toward the subject eye and changing the optical characteristics of the visual target light flux. As the subjective optical characteristics of the subject eye, at least one of the refractive power (spherical power, cylindrical power, astigmatism axis angle, and the like), contrast sensitivity, binocular vision function (for example, the amount of phoria and stereoscopic vision function), and the like may be measured.

The subjective measurement means may include a light projecting optical system (for example, light projecting optical system 30). The light projecting optical system projects a visual target light flux toward the subject eye. The light projecting optical system projects the visual target light flux emitted from the visual target presenting means toward the subject eye. Further, the light projecting optical system may project the visual target light flux emitted from the visual target presenting means toward the subject eye and corrected through the correction optical system. For example, as the visual target presenting means, a display means (for example, display 31) may be used.

Further, the subjective measurement optical system may include a correction optical system (for example, correction optical system 60). The correction optical system is arranged in an optical path of the light projecting optical system and changes the optical characteristics of the visual target light flux. The correction optical system may have a configuration in which the optical characteristics of the visual target light flux can be changed. For example, the correction optical system may be able to change at least one of the spherical power, the cylindrical power, the astigmatism axis angle, and the like of the visual target light flux by controlling the optical element. Further, for example, the correction optical system may correct the spherical power of the subject eye by optically changing the presentation position (presentation distance) of the visual target with respect to the subject eye. In this case, the visual target presenting means may be moved in an optical axis direction, or an optical element (for example, a spherical lens or the like) arranged in the optical path may be moved in the optical axis direction. Further, for example, the correction optical system may be a refractive power measurement unit (phoropter) in which an optical element is arranged in front of the subject eye. Further, for example, the correction optical system may change the optical characteristics of the visual target light flux by arranging an optical element between the visual target presenting means and the optical member for guiding the visual target light flux from the light projecting optical system toward the subject eye, and by controlling the optical element. In other words, the correction optical system may be configured as a phantom lens refractometer (phantom correction optical system).

In addition, in the present embodiment, the information analyzing device may receive the objective value of the subject eye in the objective examination performed, for the subject eye, by using a device different from the information analyzing device, and the data log related to the objective examination, to acquire these. In addition, in the present embodiment, the information analyzing device may acquire the objective value of the subject eye in the objective examination performed, for the subject eye, by using the information analyzing device, and the data log related to the objective examination. In this case, the information analyzing device can acquire the objective value and the data log by providing the following objective measurement means.

### <Objective measurement means>

The information analyzing device may include an objective measurement means (for example, objective measurement optical system 10). The objective measurement means objectively measures the optical characteristics of the subject eye by projecting a measured light flux onto the fundus of the subject eye and receiving a fundus reflected light flux which is the measured light flux reflected by the fundus of the subject eye. As the objective optical characteristics of the subject eye, at least one of refractive power (spherical power, cylindrical power, astigmatism axis angle, and the like), an eye axial length, a corneal shape, and the like may be measured.

The objective measurement means may include a light projecting optical system (for example, light projecting optical system 10a). The light projecting optical system projects the measured light flux onto the fundus of the subject eye. In addition, the objective measurement means may include a light receiving optical system (for example, light receiving optical system 10b). The light receiving optical system receives the fundus reflected light flux which is the measured light flux reflected by the fundus of the subject eye, by a detector (for example, imaging element 22).

The information analyzing device in the present embodiment may be configured to include at least the subjective measurement means. In other words, the information analyzing device may be configured to include the subjective measurement means or to include both the subjective measurement means and the objective measurement means.

The present disclosure is not limited to the device described in the present embodiment. For example, terminal control software (program) that performs functions of the embodiment described below may be supplied to the system or device via a network or various storage media, and control device (for example, CPU or the like) of the system or device may read and execute the program.

### <Example>

An example of an information analyzing device in the present embodiment will be described.

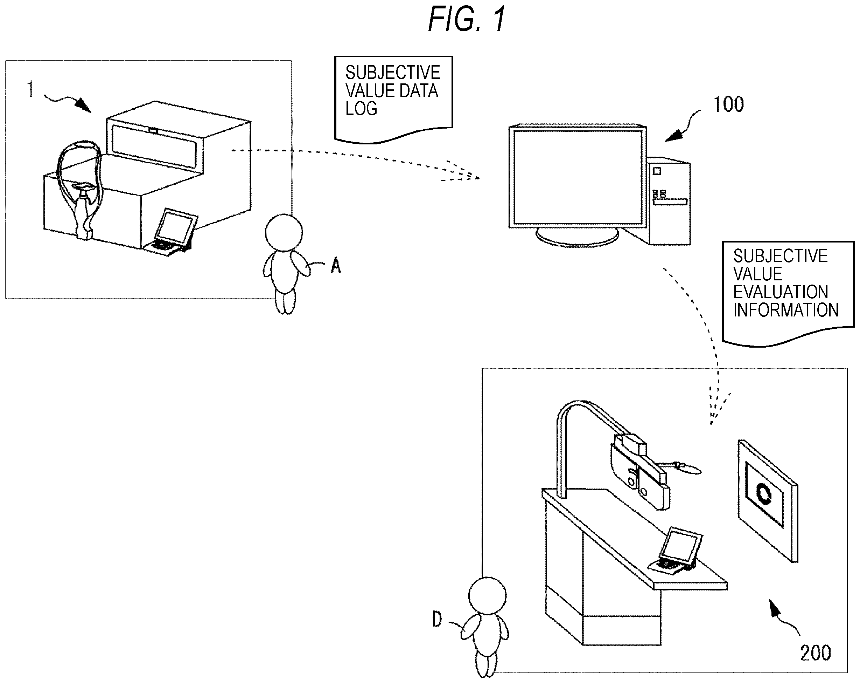

First, a subjective examination on a subject eye using the information analyzing device will be described. FIG. 1 is a view showing an example of a flow of the subjective examination on a subject eye E. For example, in the subjective examination (subjective measurement), an optometry (measurement) for the subject eye E using a first subjective optometry device 1 is performed by an assistant A who assists an examiner D. The assistant A may be unfamiliar with optometry. For example, the assistant A presents an examination target to the subject eye E, switches the correction power for correcting the subject eye E, and obtains a subjective value of the subject eye E. A control unit of the first subjective optometry device 1 transmits a subjective value of the subject eye E and a data log related to the subjective examination of the subject eye E (data log in a case where the subject eye E is examined) to an information analyzing device 100.

A control unit of the information analyzing device 100 receives and acquires the subjective value of the subject eye E and the data log related to the subjective examination of the subject eye E. Further, the control unit of the information analyzing device 100 analyzes the data log related to the subjective examination of the subject eye E, and acquires evaluation information for evaluating the reliability of the subjective examination by the assistant A. For example, the evaluation value details will be described later for the subjective examination by the assistant A may be acquired based on whether or not a predetermined data log is included. The control unit of the information analyzing device 100 transmits the subjective value of the subject eye E and the evaluation information for the subjective examination by the assistant A to a second subjective optometry device 200.

A control unit of the second subjective optometry device 200 receives and acquires the subjective value of the subject eye E and the evaluation information for the subjective examination by the assistant A. In addition, the control unit of the second subjective optometry device 200 displays the subjective value of the subject eye E and the evaluation information for the subjective examination by the assistant A on a monitor.

For example, in the subjective examination, the optometry for the subject eye E using the second subj ective optometry device 200 is operated by the examiner D, who is familiar with the optometry. It is difficult for the examiner D to determine the suitability of the subjective examination by the assistant A only by confirming the subjective value of the subject eye E, but by confirming the evaluation information together with the subjective value of the subject eye E, it becomes easier to determine the suitability of the subjective examination by the assistant A. Therefore, the examiner D can easily determine whether or not to perform a reexamination on the subject eye E, what kind of prescription should be given to the subject eye E, and the like by using the second subjective optometry device 200, and perform the optometry for the subject eye E.

In FIG. 1, the first subjective optometry device 1 and the information analyzing device 100 are different devices, but the present invention is not limited thereto. In other words, the information analyzing device 100 has a configuration including at least a receiving unit for receiving the subjective value of the subject eye E and the data log related to the subjective examination, a control unit for acquiring the evaluation information for the subjective examination by the assistant A, and a transmitting unit for outputting the subjective value of the subject eye E and the evaluation information for the subjective examination by the assistant A, has been described as an example, but the present invention is not limited thereto. For example, the first subjective optometry device 1 and the information analyzing device 100 may be used in combination. In this case, the subjective value of the subject eye E may be acquired by using the first subjective optometry device 1, and the control unit of the first subjective optometry device 1 may acquire the evaluation information for the subjective examination by the assistant A, based on the data log in a case where the subjective value of the subject eye E is acquired. For example, the control unit of the first subjective optometry device 1 may display the subjective value and the evaluation information on the monitor of the first subjective optometry device 1, or may transmit the subjective value and the evaluation information to the second subjective optometry device 200.

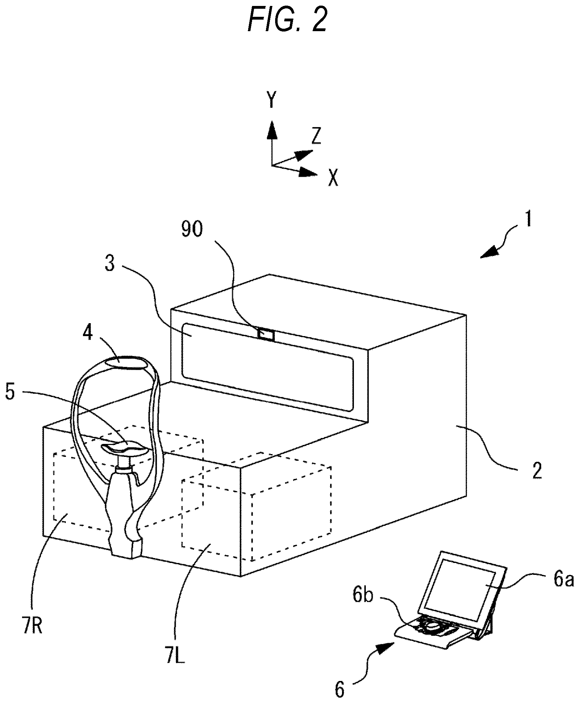

In the present example, the details will be described by taking as an example a configuration in which the first subjective optometry device 1 (hereinafter referred to as an optometry device 1) also serves as the information analyzing device 100. FIG. 2 is an external view of the optometry device 1. For example, the optometry device 1 includes a housing 2, a presentation window 3, a forehead pad 4, a chin support 5, a controller 6, an imaging unit 90, and the like.

The housing 2 has a measurement unit 7, a deflection mirror 81, a reflection mirror 84, a concave mirror 85, and the like, which will be described later, therein. The presentation window 3 is used to present a visual target to the subject eye E. The forehead pad 4 is used to keep a distance between the subject eye E and the optometry device 1 constant. The chin support 5 is used to keep a distance between the subject eye E and the optometry device 1 constant.

The controller 6 includes a monitor 6a, a switch unit 6b, and the like. The monitor 6a displays various information (for example, a measurement result of the subject eye E). The monitor 6a is a touch panel, and the monitor 6a also functions as the switch unit 6b. The switch unit 6b is used to make various settings (for example, input of a start signal). A signal corresponding to an operation instruction from the controller 6 is output to the control unit 70 by wired communication through a cable which is not shown. Further, the signal corresponding to the operation instruction from the controller 6 may be output to the control unit 70 by wireless communication through infrared rays or the like.

The imaging unit 90 includes an imaging optical system which is not shown. For example, the imaging optical system is used to image a face of an examinee. For example, the imaging optical system may be configured with an imaging element and a lens.

### <Measurement unit>

The measurement unit 7 includes a left eye measurement unit 7L and a right eye measurement unit 7R. In the present example, the left eye measurement unit 7L and the right eye measurement unit 7R include the same members. It is needless to say that at least a part of the left eye measurement unit 7L and the right eye measurement unit 7R are configured with different members. The measurement unit 7 has a pair of left and right subjective measurement units which will be described later and a pair of left and right objective measurement units which will be described later. A visual target light flux and a measured light flux from the measurement unit 7 are guided to the subject eye E through the presentation window 3.

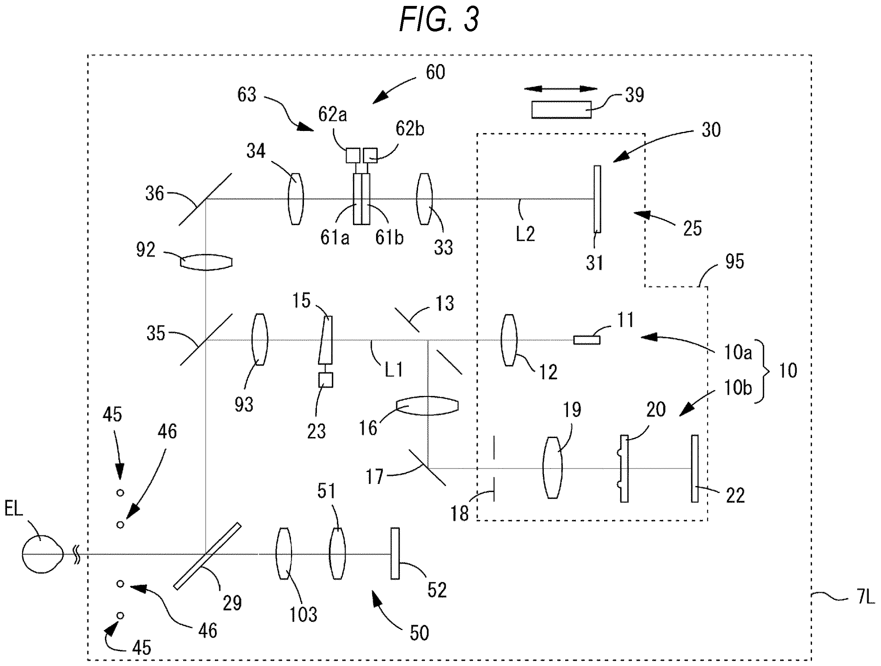

FIG. 3 is a view showing the measurement unit 7. In FIG. 3, as the measurement unit 7, the left eye measurement unit 7L is taken as an example. Since the right eye measurement unit 7R has the same configuration as that of the left eye measurement unit 7L, the description thereof will be omitted. For example, the left eye measurement unit 7L includes an objective measurement optical system 10, a subjective measurement optical system 25, a first index projection optical system 45, a second index projection optical system 46, an observation optical system 50, and the like.

### <Objective measurement optical system>

The objective measurement optical system 10 is used as a part of the configuration of the objective measurement unit that objectively measures the optical characteristics of the subject eye. In the present example, as the optical characteristics of the subject eye E, an objective measurement unit for measuring the refractive power of the subject eye E will be described as an example. In addition to the refractive power, the optical characteristics of the subject eye E may be the eye axial length, the corneal shape, and the like. For example, the objective measurement optical system 10 includes a light projecting optical system 10a and a light receiving optical system 10b.

The light projecting optical system 10a projects a spot-shaped measurement index on the fundus of the subject eye E through the pupil center part of the subject eye E. For example, the light projecting optical system 10a includes a light source 11, a relay lens 12, a hole mirror 13, a prism 15, an objective lens 93, a dichroic mirror 35, a dichroic mirror 29, and the like.

The light receiving optical system 10b takes out the fundus reflected light flux reflected by the fundus of the subject eye E in a ring shape through the peripheral portion of the pupil of the subject eye E. For example, the light receiving optical system 10b includes the dichroic mirror 29, the dichroic mirror 35, the objective lens 93, the prism 15, the hole mirror 13, a relay lens 16, a mirror 17, a light receiving diaphragm 18, a collimator lens 19, a ring lens 20, an imaging element 22, and the like.

In the present example, the description of the light projecting optical system 10a and the light receiving optical system 10b will be omitted. For details of these, refer to, for example, JP-A-2018-047049.

### <Subjective measurement optical system>

The subjective measurement optical system 25 is used as a part of the configuration of the subjective measurement unit that subjectively measures the optical characteristics of the subject eye E. In the present example, as the optical characteristics of the subject eye E, a subjective measurement unit for measuring the refractive power of the subject eye E is taken as an example. In addition to the refractive power, the optical characteristics of the subject eye E may be the contrast sensitivity, the binocular vision function (for example, the amount of phoria and the stereoscopic vision function), and the like. For example, the subjective measurement optical system 25 includes a light projecting optical system 30 and a correction optical system 60.

### <Light projecting optical system>

The light projecting optical system 30 projects a visual target light flux toward the subject eye E. For example, the light projecting optical system 30 includes the display 31, a projective lens 33, a projective lens 34, a reflection mirror 36, an objective lens 92, the dichroic mirror 35, the dichroic mirror 29, and the like.

A visual target (fixation target, examination target, and the like) is displayed on the display 31. The visual target light flux emitted from the display 31 is projected onto the subject eye E through the optical members from the projective lens 33 to the dichroic mirror 29 in order. The dichroic mirror 35 makes the optical path of the objective measurement optical system 10 and the optical path of the subjective measurement optical system 25 a common optical path. In other words, the dichroic mirror 35 makes an optical axis L1 of the objective measurement optical system 10 and an optical axis L2 of the subjective measurement optical system 25 coaxial with each other. The dichroic mirror 29 is an optical path branching member. The dichroic mirror 29 reflects the visual target light flux projected by the light projecting optical system 30 and the visual target light flux projected by the light projecting optical system 10a, which will be described later, and guides the visual target light flux and the measured light flux to the subject eye E.

### <Correction optical system>

The correction optical system 60 is arranged in the optical path of the light projecting optical system 30. Further, the correction optical system 60 changes the optical characteristics of the visual target light flux emitted from the display 31. For example, the correction optical system 60 includes an astigmatism correction optical system 63, a drive mechanism 39, which will be described later, and the like.

The astigmatism correction optical system 63 is used to correct cylindrical power and an astigmatism axis angle of the subject eye E. The astigmatism correction optical system 63 is arranged between the projective lens 33 and the projective lens 34. The astigmatism correction optical system 63 includes two positive lenses, a cylindrical lens 61a and a cylindrical lens 61b, having the same focal length. Each of the cylindrical lens 61a and the cylindrical lens 61b rotates independently around the optical axis L2 by driving a rotation mechanism 62a and a rotation mechanism 62b.

In the present example, a configuration in which the cylindrical lens 61a and the cylindrical lens 61b are used as the astigmatism correction optical system 63 has been described as an example, but the present invention is not limited thereto. The astigmatism correction optical system 63 may have a configuration capable of correcting the cylindrical power, the astigmatism axis angle, and the like. As an example, the corrective lens may be taken in and out of the optical path of the light projecting optical system 30.

### <Driving unit>

In the present example, the light source 11 and the relay lens 12 included in the light projecting optical system 10a, the light receiving diaphragm 18, the collimator lens 19, a ring lens 20, the imaging element 22 included in the light receiving optical system 10b, and the display 31 included in the light projecting optical system 30 are configured to be integrally moved in the optical axis direction by the drive mechanism 39. In other words, the display 31, the light source 11, the relay lens 12, the light receiving diaphragm 18, the collimator lens 19, the ring lens 20, and the imaging element 22 are synchronized as a driving unit 95 and are integrally moved by the drive mechanism 39. The drive mechanism 39 includes a motor and a slide mechanism. A moving position where the drive mechanism 39 has moved is detected by a potentiometer which is not shown.

The drive mechanism 39 moves the display 31 in the optical axis L2 direction by moving the driving unit 95 in the optical axis direction. Accordingly, in the objective measurement, fog can be applied to the subject eye E. In the subjective measurement, a presentation distance of the visual target to the subject eye E can be optically changed to correct the spherical power of the subject eye E. In other words, the configuration in which the display 31 is moved in the direction of the optical axis L2 is used as a spherical correction optical system for correcting the spherical power of the subject eye E, and the spherical power of the subject eye E is corrected by changing the position of the display 31. The configuration of the spherical correction optical system may be different from that of the present example. For example, the spherical power may be corrected by arranging multiple optical elements in the optical path. Further, for example, the spherical power may be corrected by arranging the lens in the optical path and by moving the lens in the optical axis direction.

Further, the drive mechanism 39 moves the light source 11, the relay lens 12, and the members from the light receiving diaphragm 18 to the imaging element 22 in the optical axis L1 direction by moving the driving unit 95 in the optical axis direction. Accordingly, the light source 11, the light receiving diaphragm 18, and the imaging element 22 are arranged to be optically conjugated with respect to the fundus of the subject eye E. Regardless of the movement of the driving unit 95, the hole mirror 13 and the ring lens 20 are arranged to be conjugated with the pupil of the subject eye E at a constant magnification. Therefore, the fundus reflected light flux which is reflection of the measured light flux of the light projecting optical system 10a is always incident on the ring lens 20 of the light receiving optical system 10b as a parallel light flux, and a ring-shaped light flux having the same size as the ring lens 20 is imaged on the imaging element 22 in a focused state regardless of the refractive power of the subject eye E.

### <First index projection optical system and second index projection optical system>

The first index projection optical system 45 and the second index projection optical system 46 are arranged between the dichroic mirror 29 and the deflection mirror 81, which will be described later. The first index projection optical system 45 emits near infrared light for projecting an infinite-distance alignment index onto the cornea of the subject eye E. The second index projection optical system 46 is arranged at a position different from that of the first index projection optical system 45, and emits near infrared light for projecting a finite-distance alignment index on the cornea of the subject eye. The near infrared light (alignment light) emitted from the second index projection optical system 46 is also used as anterior eye part photographing light for photographing an anterior eye part of the subject eye by the observation optical system 50.

### <Observation optical system>

The observation optical system (imaging optical system) 50 includes the dichroic mirror 29, an objective lens 103, an imaging lens 51, an imaging element 52, and the like. The dichroic mirror 29 transmits the anterior eye part observation light and the alignment light. The imaging element 52 has an imaging surface arranged at a position conjugated with the anterior eye part of the subject eye E. The output from the imaging element 52 is input to the control unit 70. Accordingly, the image of the anterior eye part of the subject eye E is captured by the imaging element 52 and displayed on the monitor 6a. The observation optical system 50 also serves as an optical system for detecting an alignment index image formed on the cornea of the subject eye E by the first index projection optical system 45 and the second index projection optical system 46, and the position of the alignment index image is detected by the control unit 70.

### <Internal configuration of information analyzing device>

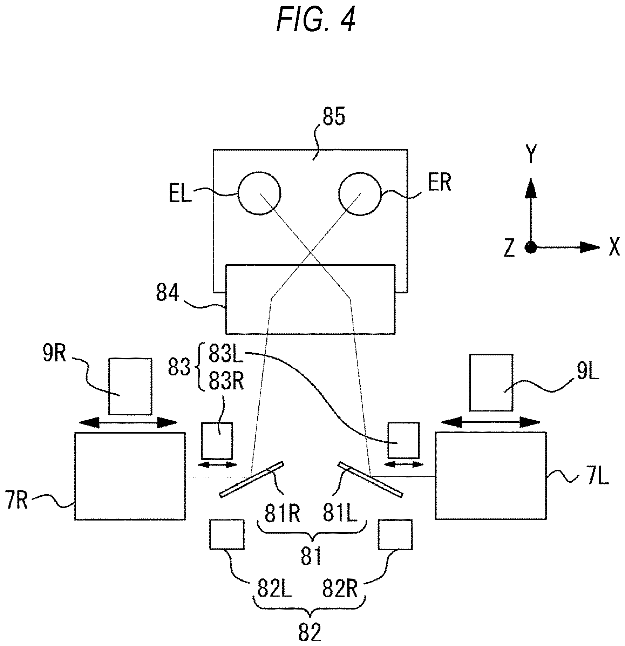

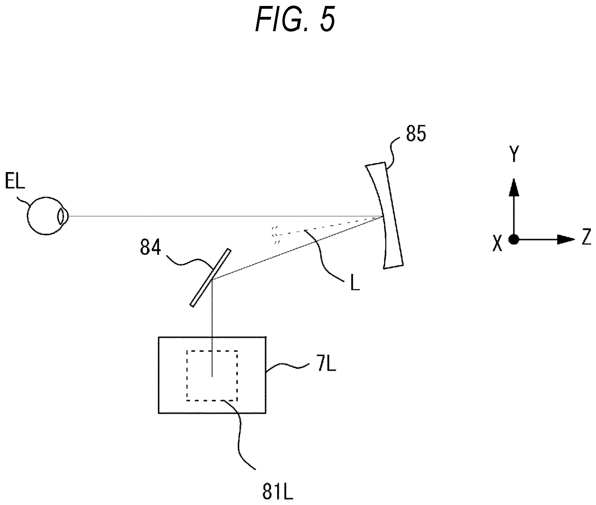

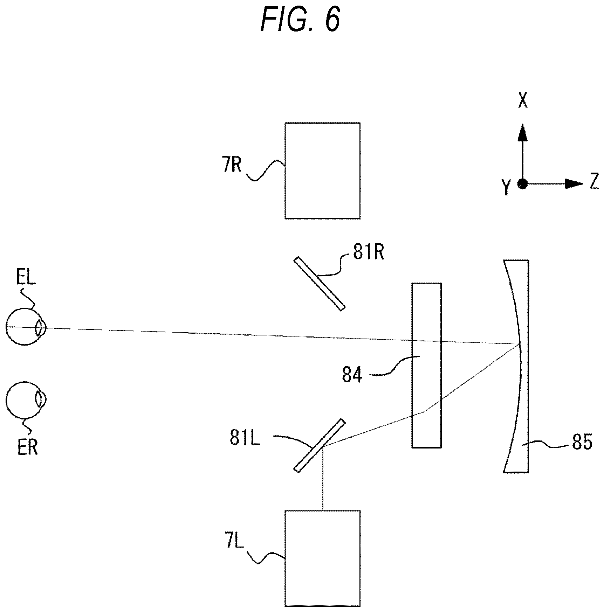

Next, the internal configuration of the optometry device 1 will be described. FIG. 4 is a schematic configuration view of the inside of the optometry device 1 when viewed from the front. FIG. 5 is a schematic configuration view of the inside of the optometry device 1 when viewed from the side. FIG. 6 is a schematic configuration view of the inside of the optometry device 1 when viewed from above. FIGs. 5 and 6 show only the optical axis of the left eye measurement unit 7L, for convenience of description.

The optometry device 1 includes the subjective measurement unit. For example, the subjective measurement unit includes the measurement unit 7, the deflection mirror 81, a drive mechanism 82, a driving unit 83, a reflection mirror 84, a concave mirror 85, and the like. The subjective measurement unit is not limited to this configuration. For example, the configuration may not include the reflection mirror 84. In this case, the visual target light flux from the measurement unit 7 may be emitted in an oblique direction with respect to the optical axis L of the concave mirror 85 after passing through the deflection mirror 81. Further, for example, the configuration may include a half mirror. In this case, the visual target light flux from the measurement unit 7 may be emitted in the oblique direction with respect to the optical axis L of the concave mirror 85 through the half mirror, and the reflected light flux may be guided to the subject eye E.

The optometry device 1 has a left eye driving unit 9L and a right eye driving unit 9R, and the left eye driving unit 9L and the right eye driving unit 9R can move the left eye measurement unit 7L and the right eye measurement unit 7R in the X direction, respectively. For example, by moving the left eye measurement unit 7L and the right eye measurement unit 7R, the distance between the measurement unit 7 and the deflection mirror 81, which will be described later, changes, and the presentation position of the visual target light flux from the measurement unit 7 in the Z direction is changed. Accordingly, the measurement unit 7 is adjusted in the Z direction to guide the visual target light flux corrected by the correction optical system 60 to the subject eye E and to form an image of the visual target light flux corrected by the correction optical system 60 on the fundus of the subject eye E.

For example, the deflection mirror 81 has a right eye deflection mirror 81R and a left eye deflection mirror 81L provided in left and right pair. For example, the deflection mirror 81 is arranged between the correction optical system 60 and the subject eye E. In other words, the correction optical system 60 in the present example has a left eye correction optical system and a right eye correction optical system provided in left and right pair, the left eye deflection mirror 81L is arranged between the left eye correction optical system and a left eye EL, and the right eye deflection mirror 81R is arranged between the right eye correction optical system and a right eye ER. For example, the deflection mirror 81 is preferably arranged at the pupil conjugate position.

For example, the left eye deflection mirror 81L reflects the light flux projected from the left eye measurement unit 7L and guides the light flux to the left eye EL. Further, for example, the left eye deflection mirror 81L reflects the fundus reflected light flux from the left eye EL and guides the fundus reflected light flux to the left eye measurement unit 7L. For example, the right eye deflection mirror 81R reflects the light flux projected from the right eye measurement unit 7R and guides the light flux to the right eye ER. Further, for example, the right eye deflection mirror 81R reflects the fundus reflected light flux from the right eye ER and guides the fundus reflected light flux to the right eye measurement unit 7R. In the present example, a configuration in which a deflection mirror 81 is used as a deflection member that reflects the light flux projected from the measurement unit 7 and guides the light flux to the subject eye E is described as an example, but the present invention is not limited thereto. The deflection member may be, for example, a prism, a lens, or the like, as long as the deflection member can reflect the light flux projected from the measurement unit 7 and guide the light flux to the subject eye E.

For example, the drive mechanism 82 includes a motor (driving unit) and the like. For example, the drive mechanism 82 includes a drive mechanism 82L for driving the left eye deflection mirror 81L and a drive mechanism 82R for driving the right eye deflection mirror 81R. For example, the deflection mirror 81 rotates and moves by driving the drive mechanism 82. For example, the drive mechanism 82 rotates the deflection mirror 81 with respect to the rotation axis in the horizontal direction (X direction) and the rotation axis in the vertical direction (Y direction). In other words, the drive mechanism 82 rotates the deflection mirror 81 in the XY directions. The rotation of the deflection mirror 81 may be in either the horizontal direction or the vertical direction.

For example, the driving unit 83 includes a motor or the like. For example, the driving unit 83 includes a driving unit 83L for driving the left eye deflection mirror 81L and a driving unit 83R for driving the right eye deflection mirror 81R. For example, the deflection mirror 81 moves in the X direction by driving the driving unit 83. For example, by moving the left eye deflection mirror 81L and the right eye deflection mirror 81R, a distance between the left eye deflection mirror 81L and the right eye deflection mirror 81R is changed, and the distance in the X direction between the left eye optical path and the right eye optical path can be changed according to a distance between the pupils of the subject eyes E.

For example, a plurality of deflection mirrors 81 may be provided in each of the left eye optical path and the right eye optical path. For example, a configuration in which two deflection mirrors are provided in each of the left eye optical path and the right eye optical path (for example, a configuration in which two deflection mirrors are provided in the left eye optical path) can be described. In this case, one deflection mirror may be rotated in the X direction and the other deflection mirror may be rotated in the Y direction. For example, by rotating and moving the deflection mirror 81, an apparent light flux for forming an image of the visual target light flux in front of the subject eye is deflected, and the formation position of the image of the visual target light flux can be optically corrected.

For example, the concave mirror 85 is shared by the left eye measurement unit 7L and the right eye measurement unit 7R. For example, the concave mirror 85 is shared by the left eye optical path including the left eye correction optical system and the right eye optical path including the right eye correction optical system. In other words, the concave mirror 85 is arranged at a position that passes through both the left eye optical path including the left eye correction optical system and the right eye optical path including the right eye correction optical system. It is needless to say that the concave mirror 85 may not be configured to be shared by the left eye optical path and the right eye optical path. In other words, a concave mirror may be provided in each of the left eye optical path including the left eye correction optical system and the right eye optical path including the right eye correction optical system. For example, the concave mirror 85 guides the visual target light flux that has passed through the correction optical system 60 to the subject eye E, and forms an image of the visual target light flux that has passed through the correction optical system 60 in front of the subject eye E.

### <Optical path of subj ective measurement unit>

The optical path of the subjective measurement unit will be described by taking the left eye optical path as an example. The right eye optical path has the same configuration as that of the left eye optical path. For example, in the subjective measurement unit for the left eye, in a case where the visual target light flux emitted from the display 31 in the subjective measurement optical system 25 is incident on the astigmatism correction optical system 63 through the projective lens 33, and passes through the astigmatism correction optical system 63, the visual target light flux is guided toward the left eye deflection mirror 81L from the left eye measurement unit 7L through the projective lens 34, the reflection mirror 36, the objective lens 92, the dichroic mirror 35, and the dichroic mirror 29. The visual target light flux reflected by the left eye deflection mirror 81L is reflected by the reflection mirror 84 toward the concave mirror 85. The visual target light flux emitted from the display 31 reaches the left eye EL through each optical member in this manner.

Accordingly, an image of the visual target light flux corrected by the correction optical system 60 is formed on the fundus of the left eye EL with reference to the spectacles wearing position of the left eye EL (for example, approximately 12 mm from the corneal apex position). Therefore, the accommodation of the spherical power by the spherical power correction optical system (in the present example, the drive of the drive mechanism 39) in front of the eyes, is equivalent to the fact that the astigmatism correction optical system 63 is arranged in front of the eye. In a natural state, the examinee can collimate the image of the visual target light flux formed in front of the eye optically at a predetermined examination distance through the concave mirror 85.

### <Control unit>

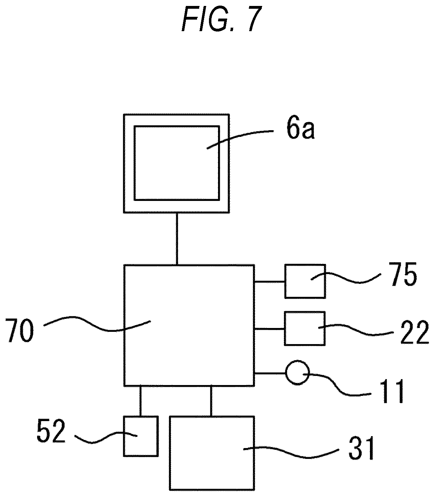

FIG. 7 is a view showing a control system of the optometry device 1. For example, various members such as the monitor 6a, a non-volatile memory 75 (hereinafter referred to as a memory 75), the light source 11, the imaging element 22, the display 31, and the imaging element 52 included in the measurement unit 7, and the like are electrically connected to the control unit 70. Further, for example, the control unit 70 is electrically connected to a driving unit which is not shown provided with the driving unit 9, the drive mechanism 39, the driving unit 83, and the like.

For example, the control unit 70 includes a CPU (processor), a RAM, a ROM, and the like. For example, the CPU controls each member of the optometry device 1. For example, the RAM temporarily stores various pieces of information. For example, various programs for controlling the operation of the optometry device 1, the visual target, initial values, and the like are stored in the ROM. The control unit 70 may include a plurality of control units (that is, a plurality of processors).

For example, the memory 75 is a non-transitory storage medium that can hold stored contents even when power supply is interrupted. For example, as the memory 75, a hard disk drive, a flash ROM, a USB memory, or the like can be used. For example, the memory 75 stores a control program for controlling the objective measurement unit and the subjective measurement unit.

### <Control operation>

The control operation of the optometry device 1 will be described.

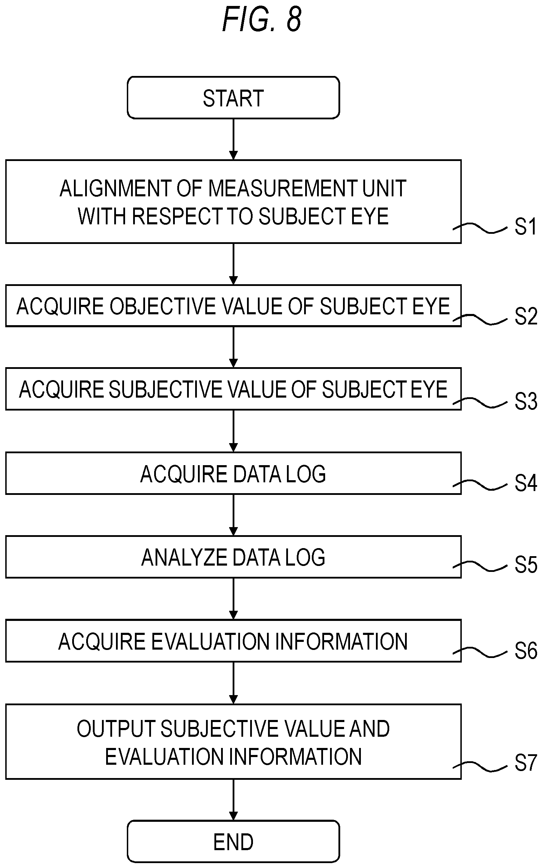

FIG. 8 is an example of a flowchart showing a control operation of the optometry device 1. For example, in the present example, the assistant A who is unfamiliar with optometry performs the optometry for the subject eye E using the optometry device 1 according to this flowchart.

### <Alignment of measurement unit with respect to subject eye (S1)>

The assistant A instructs the examinee to place the chin on the chin support 5 and observe the presentation window 3. Further, the assistant A operates the switch unit 6b to select a fixation target for fixing the subject eye E. The control unit 70 causes the display 31 to display the fixation target in response to an input signal from the switch unit 6b. By projecting the visual target light flux from the display 31 onto the subject eye E, the fixation target is presented in front of the eye.

Subsequently, the assistant A operates the switch unit 6b to select a switch for starting the alignment between the subject eye E and the measurement unit 7. The control unit 70 projects an alignment index image by the first index projection optical system 45 and the second index projection optical system 46 onto the cornea of the subject eye E, in response to the input signal from the switch unit 6b. Further, the control unit 70 detects a deviation of the measurement unit 7 in the X direction, the Y direction, and the Z direction with respect to the subject eye E by using the alignment index image, and moves the measurement unit 7 based on this deviation. Accordingly, the alignment is completed.

### <Acquisition of objective value (S2)>

When the alignment of the measurement unit 7 with respect to the subject eye E is completed, the assistant A starts the obj ective measurement for the subject eye E. For example, the objective measurements are sequentially performed on the left eye EL and the right eye ER, and the objective value of the left eye EL and the objective value of the right eye ER are acquired, respectively. In addition, the objective measurements may be performed on the left eye EL and the right eye ER at the same time, and the obj ective value of the left eye EL and the obj ective value of the right eye ER may be acquired at the same time.

The assistant A operates the switch unit 6b to select a predetermined fixation target (for example, a landscape visual target). The control unit 70 causes the display 31 to display the fixation target in response to the input signal from the switch unit 6b. First, the assistant A performs a preliminary measurement of the objective value for the subject eye E. The control unit 70 moves the display 31 in the optical axis L2 direction according to the result of the preliminary measurement, and fogs the subject eye E. In other words, the control unit 70 once moves the display 31 to a position where the subject eye E is in focus, then moves the display 31 to a position where amount of fog is appropriate, and fogs the subject eye E. Then, the assistant A performs the main measurement of the objective value for the subject eye E. The control unit 70 irradiates the fundus of the subject eye E with the measured light flux from the light source 11, and causes the imaging element 22 to image the fundus reflected light flux reflected by the fundus as a ring image. The control unit 70 calculates the objective optical characteristics of the subject eye E based on the ring image. As a result, the objective value of the subject eye E is acquired.

### <Acquisition of subjective value (S3)>

When the objective value of the subject eye E is obtained, the assistant A starts the subjective measurement for the subject eye E. For example, the subjective measurements are sequentially performed on the left eye EL and the right eye ER, and the subjective value of the left eye EL and the subj ective value of the right eye ER are acquired, respectively. In addition, the subjective measurements may be performed on the left eye EL and the right eye ER at the same time, and the subjective value of the left eye EL and the subjective value of the right eye ER may be acquired at the same time.

The assistant A operates the switch unit 6b to select a predetermined examination target (for example, a Landolt ring visual target). The control unit 70 causes the display 31 to display the examination target in response to the input signal from the switch unit 6b. Subsequently, the assistant A operates the switch unit 6b to set a desired correction power. For example, the assistant A may set a desired correction power based on the objective value of the subject eye E. The control unit 70 controls at least one of the light projecting optical system 30 and the correction optical system 60 according to the input signal from the switch unit 6b. For example, the control unit 70 may correct the spherical power of the subject eye E by driving the drive mechanism 39 and moving the display 31 in the optical axis L2 direction. Further, for example, the control unit 70 drives the rotation mechanism 62a and the rotation mechanism 62b to rotate the cylindrical lens 61a and the cylindrical lens 61b around the optical axis L2b, and at least one of the cylindrical power and the astigmatism axis angle of the subject eye E may be corrected. As a result, the degree of refraction of the subject eye E is corrected by a predetermined diopter value (for example, 0 D or the like).

The assistant A operates the switch unit 6b to confirm whether the correction power for correcting the subject eye E is appropriate while switching the visual acuity value of the examination target presented to the subject eye E. When the correction power to correct the subject eye E is inappropriate, the degree of refraction of the subject eye E may be corrected with a diopter value different from the predetermined diopter value, and it may be confirmed again whether the correction power is appropriate. For example, at this time, based on the input signal from the switch unit 6b, a data log indicating the visual acuity value of the examination target switched by the assistant A, the correction power switched by the assistant A, and the like are sequentially stored (accumulated) in the memory 75.

For example, accordingly, the assistant A determines that the weakest correction power at which the highest degree of visual acuity of the subject eye E is obtained is the completely corrected value of the subject eye E. The control unit 70 acquires a completely corrected value as a subjective value of the subject eye E and stores the acquired value in the memory 75.

### <Acquisition of data log (S4)>

The control unit 70 sequentially stores data logs related to the objective measurement and the subjective measurement in the memory 75 while the objective measurement and the subjective measurement are being performed for the subject eye E. For example, the control unit 70 may sequentially store the data log related to the objective measurement of the subject eye E in the memory 75. In addition, for example, the control unit 70 may sequentially store the data log related to the subjective measurement of the subject eye E in the memory 75.

In the present example, as the data log related to the subjective measurement of the subject eye E, the data log related to the measurement of the spherical power of the subject eye E and the data log related to the measurement of the astigmatic power of the subject eye E are sequentially stored in the memory 75. For example, in the measurement of the spherical power and the astigmatic power of the subject eye E, the control unit 70 stores the log that can identify the operation of the assistant A, the measurement procedure of the assistant A, the state of the subject eye E, the measured completely corrected value of the subject eye E, and the like, in the memory 75.

The data log related to the measurement of the spherical power of the subject eye E may include a data log related to the measurement of the accommodation amount of the subject eye E. For example, the log that can identify the operation of the assistant A, the measurement procedure of the assistant A, the state of the subject eye E, the measured accommodation amount of the subject eye E, and the like, in the measurement of the accommodation amount of the subject eye E, may be included.

### <Analyzing of data log (S5)>

The control unit 70 analyzes the data log related to the subjective measurement of the subject eye E, and detects whether or not a predetermined data log is included. In the present example, a case of acquiring the number of times of switching the correction power of the subject eye E by the assistant A, and the accommodation amount of the subject eye E by analyzing the data log related to the subjective measurement of the subject eye E and detecting whether or not the predetermined data log is included, is exemplified.

First, the number of times of switching the correction power of the subject eye E by the assistant A will be described. For example, in the subjective measurement for the subject eye E, a predetermined data log is stored in the memory 75 every time the assistant A changes the correction power. The control unit 70 extracts such a data log from the data log related to the subjective measurement of the subject eye E, and counts the number of the extracted data log. For example, the number of times of switching the spherical power by the assistant A, the number of times of switching the cylindrical power by the assistant A, the number of times of switching the astigmatism axis angle by the assistant A, and the like may be counted.

In the above, the number of times of switching the correction power by the assistant A is acquired by extracting a predetermined data log from the data log related to the subjective measurement of the subject eye E, but the present invention is not limited thereto. It is needless to say that, when the data log related to the subjective measurement of the subject eye E includes a data log indicating the number of times of switching the correction power by the assistant A, the number of times of switching the correction power can also be directly acquired based on this data log.

Next, the accommodation amount of the subject eye E will be described. For example, in the objective measurement for the subject eye E, a predetermined data log indicating the objective value of the subject eye E is stored in the memory 75. Further, for example, in the subjective measurement for the subject eye E, a predetermined data log indicating the completely corrected value of the subject eye E is stored in the memory 75. The control unit 70 extracts these data logs and obtains the difference between the objective value of the subject eye E and the completely corrected value of the subject eye E. Further, the control unit 70 determines the accommodation amount of the subject eye E according to the difference between the objective value of the subject eye E and the completely corrected value of the subject eye E. For example, the control unit 70 may determine that, as the difference between the objective value of the subject eye E and the completely corrected value of the subject eye E increases in the negative direction, the accommodation amount acting on the subject eye E increases, and as the difference between the objective value of the subject eye E and the completely corrected value of the subject eye E increases in the positive direction, the unadjusted amount of the subject eye E increases.

In the above, the accommodation amount of the subject eye E is obtained based on the difference between the objective value of the subject eye E and the completely corrected value of the subject eye E, but the present invention is not limited thereto. For example, the presence or absence of accommodation of the subject eye E may be acquired by determining whether the difference between the objective value of the subject eye E and the completely corrected value of the subject eye E is 0 D or a value different from 0 D. In other words, in a case where the difference between the objective value of the subject eye E and the completely corrected value of the subject eye E is 0 D, it may be determined that the accommodation is not working on the subject eye E. In addition, when the difference between the objective value of the subject eye E and the completely corrected value of the subject eye E is a value of negative power, it may be determined that the accommodation acts on the subject eye E. In addition, when the difference between the objective value of the subject eye E and the completely corrected value of the subject eye E is a value of positive power, it may be determined that the subject eye E was not adjusted.

### <Acquisition of evaluation information (S6)>

In the present example, the control unit 70 acquires each score (score K1 and score K2 described later) based on the number of times of switching the correction power by the above-described assistant A and the accommodation amount of the subject eye E. Further, in the present example, the control unit 70 acquires the evaluation value for evaluating the reliability of the subjective measurement by the assistant A by subtracting each score from the maximum score (score K3 described later) of the subjective measurement by assistant A.

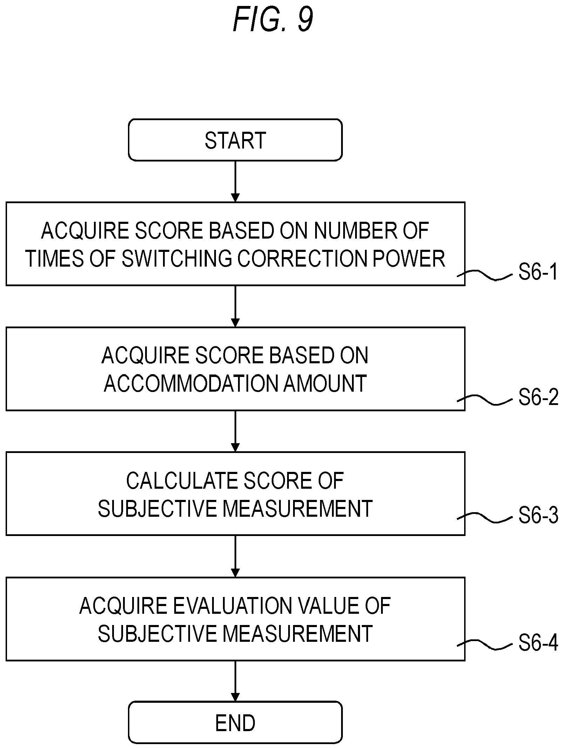

FIG. 9 is an example of a flowchart showing acquisition of the evaluation values for the subjective measurement by the assistant A. The acquisition of each score and the acquisition of the evaluation value by the control unit 70 will be described in detail.

### <Acquisition of score based on number of times of switching correction power (S6-1)>

First, a score based on the number of times of switching the correction power of the subject eye E by the assistant A is acquired. The control unit 70 acquires the score K1 based on the number of times of switching the correction power by using a table in which the score is associated with the number of times of switching the correction power. For example, such a table may be provided for each of the number of times of switching the spherical power, the number of times of switching the cylindrical power, and the number of times of switching the astigmatism axis angle. Further, for example, such a table may be preset from experiments, simulations, and the like. As an example, in the present example, the score K1 may be set to become a larger value as the number of times of switching the correction power increases. Accordingly, the control unit 70 can acquire the score K1 corresponding to the number of times of switching the correction power.

### <Acquisition of score based on accommodation amount (S6-2)>

Next, a score based on the accommodation amount of the subject eye E is acquired. The control unit 70 acquires the score K2 based on the accommodation amount of the subject eye E by using a table in which the accommodation amount of the subject eye E is associated with the score. For example, such a table may be preset from experiments, simulations, and the like. As an example, in the present example, the value of the score K2 may be finely determined according to the accommodation amount of the subject eye E. For example, the score K2 may be set to increase as the accommodation amount of the subject eye E increases. As a result, the control unit 70 can acquire the score K2 corresponding to the accommodation amount of the subject eye E.

### <Calculation of score of subjective measurement (S6-3)>

Next, the score K3 of the subj ective measurement by the assistant A is acquired. For example, the maximum value (for example, 100) may be set in advance for the score of the subjective measurement by the assistant A. The control unit 70 calculates the score K3 of the subjective measurement by the assistant A by subtracting the above-described scores K1 and K2 from the maximum value of the score of the subjective measurement.

### <Acquisition of evaluation information for subjective measurement (S6-4)>

The control unit 70 acquires the evaluation value obtained by classifying the score K3 of the subjective measurement by the assistant Abased on a predetermined reference. For example, the evaluation value may be indicated by a value from 10 to 1. In other words, the evaluation values may be expressed in 10 stages from the evaluation value 10 to the evaluation value 1. Further, for example, as for the evaluation values, a reference value (reference range) of the score K3 may be set in advance at each stage. As an example, the score K3 at the evaluation value 10 may be set to 100 to 91, the score K3 at the evaluation value 9 may be set to 90 to 81, ..., and the score K3 at the evaluation value 1 may be set to 10 to 0. For example, the control unit 70 can acquire the evaluation value for the subjective measurement by the assistant A by referring to which stage of the evaluation value the score K3 corresponds to.

The acquisition of the above score and evaluation information will be described by exemplifying specific numerical values. For example, when the number of times of switching the correction power is 5, the control unit 70 obtains the numerical values of the score K1 as - 16 by referring to the table. Further, for example, when the accommodation amount of the subject eye E is -1 D, the control unit 70 acquires the numerical value of the score K2 as -2 by referring to the table. Subsequently, for example, the control unit 70 subtracts the numerical value -16 of the score K1 and the numerical value -2 of the score K2 from 100 which is the maximum numerical value of the subjective measurement by the assistant A, and acquires the score K3 of the subjective measurement by the assistant A as 77. Further, for example, the control unit 70 compares the reference value of the score K3 provided for each evaluation value with the obtained score K3, and acquires the evaluation value 8 (the score K3 is 80 to 71) as an evaluation value for the subjective measurement by the assistant A.

In the present example, the evaluation value for the subjective measurement by the assistant A is acquired based on the score K3 of the subjective measurement by the assistant A, but the present invention is not limited thereto. It is needless to say that the score K3 of the subjective measurement by the assistant A may be acquired as an evaluation value for the subjective measurement by the assistant A.

### <Output of subjective value and evaluation information (S7)>

The control unit 70 displays both the completely corrected value of the subject eye E and the acquired evaluation value on the monitor 6a.

FIG. 7 is an example of a display screen of the monitor 6a. On the monitor 6a, a completely corrected value 110 of the subject eye E, an evaluation value 101, and a factor 102 that contributed to the evaluation value 101 (in the present example, the number of times of switching the correction power by the assistant A, the degree of accommodation of the subject eye E, and the like), the score K3, the details 103 of the score K3, and the like are displayed. On the monitor 6a, at least one of the evaluation value 101 and the factor 102 together with the completely corrected value 110 of the subject eye E may be displayed.

For example, on the monitor 6a, all the factors 102 that contributed to the evaluation value 101 may be displayed. Further, for example, on the monitor 6a, some factors 102 that contributed to the evaluation value 101 may be displayed. As an example, on the monitor 6a, only the factor 102 having a large contribution to the evaluation value 101 may be displayed. For example, in this case, the control unit 70 may set a predetermined threshold value in the detail 103 of the score for each factor and display the factor exceeding the threshold value.

For example, the examiner D can determine the suitability of the subjective examination by the assistant A by confirming the completely corrected value of the subject eye E in the subjective examination performed by the assistant A and the evaluation value 101 for evaluating the reliability of the subjective examination, and can easily determine whether to perform the reexamination on the subject eye E, an appropriate prescription for the subject eye E, and the like.

As described above, for example, the information analyzing device in the present example acquires the subjective value of the subject eye measured by the subjective examination, acquires the data log related to the subjective examination in a case where the subjective value of the subject eye is measured by the subjective examination, acquires the evaluation information for evaluating the reliability of the subjective examination, based on the data log, and outputs the subjective value and the evaluation information. As a result, the examiner can easily confirm the suitability of the subjective examination by an unfamiliar operator (assistant A), and thus more easily determine whether to perform a reexamination on the subject eye, an appropriate prescription for the subject eye, and the like.

Further, for example, the information analyzing device in the present example acquires at least one of the data log related to the spherical examination for acquiring the spherical information of the subject eye and the data log related to the astigmatism examination for acquiring the astigmatism information of the subject eye, as the data log related to the subjective examination of the subject eye. Accordingly, the examiner can obtain the evaluation information that reflects whether or not the spherical information and the astigmatism information of the subject eye is appropriately examined, and can easily determine reexamined or prescription for the subject eye. Further, for example, the information analyzing device in the present example acquires at least the data log related to the accommodation examination for acquiring the accommodation information of the subject eye, as the data log related to the spherical examination of the subject eye. Accordingly, accordingly, the examiner can obtain the evaluation information reflecting the accommodation information of the subject eye, and can perform an appropriate examination and prescription for the subj ect eye.

Further, for example, the information analyzing device in the present example includes the subjective measurement means for subjectively measuring the optical characteristics of the subject eye by projecting the visual target light flux toward the subject eye and changing the optical characteristics of the visual target light flux, acquires the subjective value measured by the subjective measurement means, and acquires a data log in a case where the subjective value is measured by the subjective measurement means. As a result, the subjective examination on the subject eye, the analysis of data logs, and the output of the subjective values and the evaluation information can be performed in one device.

### <Modification example>

In the present example, a configuration in which the accommodation state (for example, accommodation amount) in the subjective measurement of the subject eye E is determined from the difference between the objective value of the subject eye E and the completely corrected value of the subject eye E, based on the data log related to the objective measurement and the subjective measurement of the subject eye E, to acquire the score K2 has been described as an example, but the present invention is not limited thereto. For example, during the subjective measurement of the subject eye E, the objective measurement for the subject eye E may be performed, and the accommodation state of the subject eye E during the subjective measurement may be determined based on the change over time in the objective value of the subject eye E. In this case, the control unit 70 captures and analyzes a ring image reflected on the fundus of the subject eye E at the same time as the subjective measurement for the subject eye E, and stores the change in the objective value of the subject eye E as a data log in the memory 75.

For example, the control unit 70 may analyze such a data log and detect whether the objective value of the subject eye E gradually changes to a negative value, or the objective value of the subject eye E gradually changes to a positive value. It is needless to say that, for example, the control unit 70 may analyze such a data log and detect the change rate in which the objective value of the subject eye E gradually changes to a negative value or a positive value. In this case, the control unit 70 may determine the accommodation state in the subjective measurement of the subject eye E from the change or the change rate of the objective value of the subject eye E. For example, the control unit 70 determine that, as the change in the objective value of the subject eye E increases in the positive direction, the amount of accommodation acting on the subject eye E increases, and as the change in the objective value of the subject eye E increases, the unadjusted amount of the subject eye E increases.

Further, for example, the control unit 70 may analyze such a data log and detect whether or not the change in the objective value of the subject eye E has converged (that is, whether or not the change in the objective value of the subject eye E gradually decreases). In this case, the control unit 70 may determine the accommodation state in the subjective measurement of the subject eye E from the convergence degree of the objective value of the subject eye E. For example, the control unit 70 may determine a subjective value obtained in a case where the accommodation of the subject eye E becomes constant when the change in the objective value of the subject eye E has converged, and a subjective value obtained while adjusting the subject eye E when the change in the objective value of the subject eye E has converged.

For example, the control unit 70 can acquire the score K2 by using a table in which the score is associated with such an accommodation state for the subject eye E. Further, for example, the control unit 70 may acquire the score K3 of the subjective measurement by the assistant A by subtracting the score K2 from the maximum score in the subjective measurement, and may acquire the evaluation value 101 for the subjective measurement by the assistant A by referring to which stage of the evaluation value the score K3 corresponds to.

In the present example, the data log related to the measurement of the astigmatic power of the subject eye E may include the data log related to the measurement of the residual astigmatism of the subject eye E. For example, the log that can identify the operation of the assistant A, the measurement procedure of the assistant A, the state of the subject eye E, the measured residual astigmatism of the subject eye E, and the like, in the measurement of the residual astigmatism of the subject eye E, may be included.

In this case, the control unit 70 may analyze the data log related to the measurement of the residual astigmatism of the subject eye E, and may acquire the score with respect to at least one of the degree of the residual astigmatism, the number of times of correcting the residual astigmatism, the convergence degree of the residual astigmatism, and the like in the subjective measurement of the subject eye E. For example, a table that associates the score with the degree of residual astigmatism of the subjective eye E, a table that associates the score with the number of times of correcting the residual astigmatism of the subject eye E, a table that associates the score with the convergence degree of the residual astigmatism of the subject eye E, and the like are preset, and each score can be obtained by using these tables.

Further, the control unit 70 can subtract each obtained score from the maximum value of the score of the subjective measurement by the assistant A, and can acquire the evaluation value 101 for the subj ective measurement by the assistant A by referring to which stage of the evaluation value the score K3 of the subjective measurement by the assistant A corresponds to. Accordingly, the examiner D can obtain the evaluation information reflecting the residual astigmatism information of the subject eye E, and can perform an appropriate examination and prescription for the subject eye.

In the present example, a data log stored every time the visual acuity value corresponding to the visual target visually recognized by the subject eye E changes is extracted from the data log related to the subjective measurement of the subject eye E, and the score may be acquired based on the extracted data log. For example, in this case, the control unit 70 may acquire a predetermined score according to the visual acuity value of the subject eye E in a case where the completely corrected value of the subject eye E is measured. As an example, as the visual acuity value of the subject eye E increases (as the visual acuity of the subject eye E becomes better), the value of the score subtracted from the maximum score in the subjective measurement may be set to be small, and as the visual acuity value of the subject eye E decreases (as the visual acuity of the subject eye E becomes worse), the value of the score subtracted from the maximum score in the subj ective measurement may be set to be large. The control unit 70 may subtract the obtained predetermined score from the maximum value of the score of the subjective measurement by the assistant A, and may acquire the evaluation value 101 for the subjective measurement by the assistant A by referring to which stage of the evaluation value the score K3 of the subjective measurement by the assistant A corresponds to.

In the present example, a data log indicating the optical characteristics (for example, at least one of the spherical power, the cylindrical power, and the astigmatism axis angle) of the subject eye E is extracted from the data log related to the subjective measurement of the subject eye E, and the score may be acquired based on the extracted data log. For example, in this case, when the optical characteristics of the subject eye E has a large value, a predetermined score may be subtracted from the maximum score in the subjective measurement. As an example, when the spherical power of the subject eye E has a value exceeding the range of -6 D to +6 D, the maximum score in the subjective measurement may be subtracted. In addition, as an example, when the cylindrical power of the subject eye E has a value exceeding the range of-2 D to +2 D, the maximum score in the subjective measurement may be subtracted.

In the present example, a data log indicating the state of the subject eye E (change during the examination of the examinee) is extracted from the data log related to the subjective measurement of the subject eye E, and the score may be acquired based on the extracted data log. For example, the state of the subject eye E is at least one of the states such as the number of times (frequency) of squinting the subject eye E, the number of blinks (frequency) of the subject eye E, the pupil size of the subject eye E, and whether or not the subject eye E is ptosis, and the like.

In this case, in the subj ective measurement of the subject eye E, the observation image of the anterior eye part of the subject eye E is acquired by using the observation optical system 50 or the imaging unit 90, and the state of the subject eye E determined by analyzing the pupil shape in the observation image of the anterior eye part may be sequentially stored in the memory 75 as a data log. The control unit 70 acquires a predetermined score for each state of the subject eye E, based on the data log indicating the state of the subject eye E. As an example, by using a table in which the score is associated with the number of times of squinting the eye by the examinee during the subjective measurement, the score corresponding to the number of times of squinting the subject eye E can be acquired. The control unit 70 may subtract the obtained score from the maximum value of the score of the subjective measurement by the assistant A, and may acquire the evaluation value 101 for the subjective measurement by the assistant A by referring to which stage of the evaluation value the score K3 of the subjective measurement by the assistant A corresponds to.

In the present example, an example of the configuration that acquires the score K1 based on the number of times of switching the correction power by the assistant A and the score K2 based on the degree of accommodation of the subject eye E in the subjective measurement of the subject eye E, has been described, but the present invention is not limited thereto. For example, any one of the score K1 based on the number of times of switching the correction power by the assistant A and the score K2 based on the degree of accommodation of the subject eye E may be acquired. Further, for example, the score may be acquired based on at least one of the residual astigmatism of the subject eye E, the visual acuity of the subject eye E, the state of the subject eye E, and the like as described above. Further, for example, the score may be acquired based on the objective value (for example, at least one of the objectively obtained spherical power, the cylindrical power, the astigmatism axis angle, and the higher-order aberration) of the subject eye E. It is needless to say that the score may be acquired by combining these. The control unit 70 may acquire the comprehensive evaluation value 101 for the subjective measurement of the assistant A by subtracting each obtained score from the maximum score of the subjective measurement by the assistant A.

In the present example, a configuration that subtracts each score such as the score K1 based on the number of times of switching the correction power of the score and K2 based on the degree of accommodation, from the maximum value of the score of the subjective measurement by the assistant A has been described as an example, but the present invention is not limited thereto. For example, in the present example, the average value of each score may be calculated. In this case, for example, the evaluation value may be preset with a reference value (reference range) of the average value of the scores in each stage, and the evaluation value 101 for the subjective measurement of the assistant A may be acquired by referring to which stage of the evaluation values the average value of the scores corresponds to.

In the present example, the configuration in which the evaluation value 101 of the subjective examination by the assistant A is acquired, based on the data log related to the subjective measurement of the subject eye E, and this acquired evaluation value is output has been described as an example, but the present invention is not limited thereto. For example, based on the data log related to the subjective measurement of the subject eye E, the information for prompting the reexamination of the subjective measurement for the subject eye E may be output to the examiner D. As an example, items in which a predetermined data log is detected from the data log related to the subjective measurement of the subject eye E may be displayed as a list of reexamination items. Further, as an example, for each item of the subjective measurement of the subject eye E, the item having a score exceeding a predetermined threshold value may be displayed on the monitor 6a as a list of reexamination items. At this time, the evaluation value 101, the factor 102 that contributed to the evaluation value 101, the score K3, the details 103 of the score K3, and the like may be displayed together. Accordingly, the examiner D can easily determine which item to be reexamined for the subjective measurement for the subject eye E.

Further, for example, based on the data log related to the subjective measurement of the subject eye E, information for proposing the prescription value of the subject eye E may be output to the examiner D. It is needless to say that, together with the data log related to the subjective measurement of the subject eye E, the information for proposing a prescription value may be output based on a data log indicating the results of interviews conducted with the examinee. As an example, a proposed prescription value may be acquired and output to the monitor 6a by using a mathematical model trained by a machine learning algorithm. Accordingly, the examiner D can easily determine the prescription value for the subject eye E.

The prescription value actually prescribed by the examiner D may be fed back to the control unit 70 with respect to the prescription value proposed by the control unit 70. For example, the control unit 70 may reflect newly acquired input data and output data (here, a proposed prescription value and an actually prescribed prescription value) for a mathematical model trained by a machine learning algorithm, to create a new mathematical model obtained by updating the trained mathematical model. Accordingly, the prescription value proposed by the control unit 70 may be optimized.

### REFERENCE SIGNS LIST

1 Subjective optometry device
7 Measurement unit
10 Objective measurement optical system
25 Subjective measurement optical system
70 Control unit
75 Memory
81 Deflection mirror
84 Reflection mirror
85 Concave mirror

## Claims

1. An information analyzing device that analyzes information related to a subjective examination, the information analyzing device comprising:
a subjective value acquisition means for acquiring a subjective value of a subject eye measured by the subjective examination;
a data log acquisition means for acquiring a data log related to the subjective examination in a case where the subjective value of the subject eye is measured by the subjective examination;
an evaluation information acquisition means for acquiring evaluation information for evaluating reliability of the subjective examination, based on the data log; and
an output means for outputting the subjective value and the evaluation information.

2. The information analyzing device according to claim 1,
wherein the data log acquisition means acquires at least one of a data log related to a spherical examination for acquiring spherical information of the subject eye and a data log related to an astigmatism examination for acquiring astigmatism information of the subject eye, as the data log related to the subjective examination, and
wherein the evaluation information acquisition means acquires the evaluation information based on at least one of the data log related to the spherical examination and the data log related to the astigmatism examination.

3. The information analyzing device according to claim 1 or 2,
wherein the data log acquisition means acquires at least a data log related to an accommodation examination for acquiring accommodation information of the subject eye, as the data log related to the spherical examination, and
wherein the evaluation information acquisition means acquires the evaluation information based on at least the data log related to the accommodation examination.

4. The information analyzing device according to any one of claims 1 to 3,
wherein the data log acquisition means acquires at least a data log related to a residual astigmatism examination for acquiring residual astigmatism information of the subject eye, as the data log related to the astigmatism examination, and
wherein the evaluation information acquisition means acquires the evaluation information based on at least the data log related to the residual astigmatism.

5. The information analyzing device according to any one of claims 1 to 4, further comprising:
a subjective measurement means for subjectively measuring optical characteristics of the subject eye by projecting a visual target light flux toward the subject eye and by changing optical characteristics of the visual target light flux,
wherein the subjective value acquisition means acquires the subjective value measured by the subjective measurement means, and
wherein the data log acquisition means acquires the data log in a case where the subjective value is measured by the subjective measurement means.

6. An information analyzing program used in an information analyzing device that analyzes information related to a subjective examination, the program being executed by a processor of the information analyzing device to cause the information analyzing device to execute:
a subjective value acquiring step of acquiring a subjective value obtained by measuring optical characteristics of a subject eye by the subjective examination;
a data log acquiring step of acquiring a data log related to the subjective examination; and
an evaluation information acquiring step used to confirm reliability of the subjective examination, based on the data log.
